# EUROPEAN PATENT SPECIFICATION

(11) **EP 2 843 600 B1**
(45) Date of publication and mention of the grant of the patent: **19.06.2024**
(21) Application number: 14178116.1
(22) Date of filing: 23.07.2014
(51) Int. Cl.: G06Q 50/04, A61F 13/49, G06Q 10/0639, G06Q 10/101, G06Q 30/0201, G06Q 30/0282, G05B 19/418

(54) **Method for redesigning one or more product or process parameters of a manufactured article**
Verfahren zur Umgestaltung eines oder mehrerer Produkt- oder Prozessparameter eines Artikels
Procédé de reconfiguration d'un ou plusieurs paramètres de produits ou de processus d'un article manufacturé

(30) Priority: 03.09.2013 EP 13182727
(43) Date of publication of application: 04.03.2015
(73) Proprietor: The Procter & Gamble Company, Cincinnati, OH 45202 (US)
(72) Inventor: Von den Steinen, Helen Louise, 65824 Schwalbach (DE)
(74) Representative: Elkington and Fife LLP

(56) References cited:
- US-A1- 2003 150 909
- US-A1- 2005 043 841
- US-A1- 2007 203 604
- US-A1- 2009 157 486
- US-A1- 2010 305 740

## Description

### Field of the Invention

The present invention relates to a method for redesigning one or more product or process parameters of a first manufactured article, in order to provide different product or process parameters of a second, transformed, manufactured article.

### Background of the Invention

Manufacturers strive to provide a consistent product quality to the end-users of the product. In most cases quality assurance is provided by means of process and product standards and specifications which are set by the manufacturer. Various parameters may be assigned target values, as well as upper and lower limits. If a product does not meet the required specifications, that is to say if one or more parameters tracks outside of the upper or lower limits, then the product is not released for shipment, but instead the product may be reworked, recycled or scrapped. Typically quality assurance measurements are made on an intermediate or finished product taken from the production line, and often taken to a quality assurance laboratory for the measurements to be made.

Extensive consumer research may be undertaken during the development stage of a new product in order to establish which parameters are most relevant and important to the consumer, and quality target values, and upper and lower limits, may be determined accordingly. Setting a breadth of specification between upper and lower limits which is too wide is undesirable because at least some consumers may be dissatisfied with the quality of the product. Conversely, setting a breadth of specification between upper and lower limits which is too narrow is undesirable because it increases manufacturing costs, and increases rework, recycle and scrap rates.

US 2007/0203604, published on August 30th 2007, discloses a product quality tracking system comprising a database for storing identification codes and reference numerals of a plurality of products, and for storing quality messages; a reading device; a recording module; and a statistic module for analyzing quality messages of a plurality of products having identical identification codes.

It would be desirable to provide a method that would enable consumer feedback to generate quality information which is used for refining process and product standards and specifications. US 2005/0043841 discloses systems and methods for providing alarming indications and troubleshooting indications and actions in connection with a web converting manufacturing process used for manufacturing disposable absorbent garments.

### Summary of the Invention

The present invention relates to a method for redesigning one or more process parameters of a first manufactured article, in order to provide different process parameters of a second, transformed, manufactured article, wherein the method comprises the steps of:
i) associating a unique identifier with individual first manufactured articles, or with groups of first manufactured articles;
ii) capturing and recording process data relating to the first manufactured articles;
iii) soliciting and recording consumer feedback relating to in use performance of the first manufactured articles, wherein the consumer feedback includes data relating to the separation of back ears from diapers while under tension, leakage, or absorbent article fit;
iv) correlating, by a controller, the consumer feedback with process data of a specific, individual, first manufactured article by means of the unique identifier;
v) determining different process parameters for a second manufactured article based on the correlated consumer feedback and process data for the first manufactured absorbent articles;
vi) applying, by the controller, the one or more different process parameters to a converting apparatus configured to manufacture absorbent articles; and
vii) manufacturing, by the converting apparatus, the second manufactured absorbent articles, the second manufactured absorbent articles being better adapted to meet consumer needs than the first manufactured absorbent article,
wherein each manufactured absorbent article is a diaper comprising: a topsheet; a backsheet; and an absorbent core placed between the topsheet and the backsheet to form a body of the diaper, and at least back ears which are attached to the body of the diaper in a waist region.

### Detailed Description of the Invention

### Definitions

"Consumer feedback" means any kind of information provided by the consumer as end-user which is or may be relevant to product quality data. Consumer feedback includes, for example, responses to questionnaires or interviews, complaints made by dissatisfied customers, any of which may be may be recorded in written form, or as audio or video recording; and/or images of product in use or after use. Preferably, consumer feedback is transmitted from the consumer back to the manufacturer by written communication, by verbal communication, by electronic communication, by internet interface, or combinations thereof, preferably by means of telephone; mobile devices such as mobile telephones, smartphones, tablets.

In general, a product is designed to meet certain demands and expectations of the end-user. The fulfillment, or the lack of fulfillment, of these demands and expectations is referred to herein as the "in use performance". In use performance may be measured, quantitatively and/or qualitatively, by means of data collected during use of the product. In use performance data may be collected via sensors, or by recordings made by the consumer, diaries etc.

"Product data" means data collected during the manufacture of the product. Product data may be or product quality data, preferably measurements, images, raw material data, or combinations thereof. Product data may be recorded automatically in the manufacturing process, or may be generated off-line, for example in a quality assurance laboratory.

"Process data" means data collected from the manufacturing process. Process data may be process performance data, preferably measurements, images, or combinations thereof. Process data are typically measured, using sensors in the manufacturing process for example, and recorded automatically.

"Quality control data" / "quality data standards" means manufacturing standards, parameter targets, upper and lower limits, "out of spec." limits, etc.

"Unique identifier" means, for example, serial number, bar code, QR code, etc. Preferably a unique identifier is applied to each individual product. However a unique identifier may also be applied to a bundle of individual products, or to a package containing multiple individual products. In addition, the unique identifier may be used for purposes which may or may not be linked directly with the consumer and product quality data, e.g. consumer loyalty programmes, anti-counterfeit systems, track and trace systems.

According to the present invention, quality information is generated by correlating product data and/or process data and consumer feedback, by means of the unique identifier. The unique identifier is used to associate specific, individual manufactured articles and the product data and/or process data generated from the manufacture of those articles with consumer feedback. Useful quality information can be generated from the correlation.

The quality information generated from the correlation may be evaluated with the aid of algorithms, spreadsheets, graphical interpretation, etc. Preferably, the quality information is applied to improve quality control data, quality data standards, process control strategies, process and product causal relationships, prognostics, product or process design improvements, product or process models, or combinations thereof.

In the present invention, the manufactured articles are composite products produced by a process of web converting and sequential addition of component parts - namely, absorbent articles. The term absorbent articles is used herein to refer to devices that absorb and contain body exudates, and, more specifically, to refer to devices that are placed against or in proximity to the body of the wearer to absorb and contain the various exudates discharged from the body. Absorbent articles may include diapers, training pants, adult incontinence undergarments, feminine hygiene products, and the like. In the following example the invention will be described with reference to absorbent articles.

The present disclosure relates to methods and systems for providing quality information for use in the production of manufactured articles, for example absorbent articles. More particularly, the methods and systems herein are configured with a controller to correlate manufacturing parameters and consumer feedback parameters with individual manufactured articles manufactured by a converting apparatus. The controller adjusts target manufacturing parameters on the converting apparatus based on the consumer feedback parameters and correlated manufacturing parameters. Examples of the systems herein may include a converting line adapted to produce manufactured articles, such as absorbent articles, wherein the converting line includes inspection sensors, process sensors, and a controller connected with a communication network. Inspection sensors may be configured to inspect substrates and/or component parts advancing along the converting line and communicate inspection parameters to the controller. Process sensors may be configured to monitor equipment on the converting line and communicate process parameters to the controller.

The systems herein are adapted to receive performance feedback parameters provided by consumers of the manufactured articles. The systems may further correlate inspection parameters, process parameters, and performance feedback parameters with individual manufactured articles produced on the converting line.

As discussed above, the controller may also be adapted to receive performance feedback parameters. Performance feedback parameters may be generated in various ways, as described above, and includes various information and/or data relating to the articles produced by the converting line. For example, performance parameters as set out herein are generated by consumer feedback, and may be further supplemented by other data, for example data generated as a result of laboratory testing. Performance parameters are collected as a result of soliciting and recording consumer feedback relating to in use performance of the manufactured articles. Examples of performance parameters in the field of absorbent articles include data relating to the separation of back ears from diapers while under tension; leakage; and absorbent article fit. It is to be appreciated that consumer feedback may include any kind of information provided by the consumer as end-user which is or may be relevant to product quality data. Consumer feedback includes, for example, responses to questionnaires or interviews, complaints made by dissatisfied customers, any of which may be may be recorded in written form, or as audio or video recording; and/or images of product in use or after use. Performance parameters may be transmitted directly from feedback providers to the controller in various ways, such as via written communication; electronic communication; internet interface; and/or combinations thereof. As such, performance parameters can be communicated with various types of devices, such as telephones; computers; mobile devices such as mobile telephones; smart phones; tablets; and the like. Performance parameters may also be transmitted in various ways feedback providers to intermediaries, which can then communicate and/or enter the performance parameters into the controller.

### Example

Diapers are absorbent articles which typically comprise topsheet, backsheet, and an absorbent core placed between the topsheet and the back sheet to form the body of the diaper. A diaper may further comprise front and/or back ears which are attached to the body of the diapers in the waist region. In some embodiments the ears have areas which engage, either adhesively or otherwise, with a landing zone located elsewhere on the diaper, typically also located in the waist region. The purpose of the ears is to secure the diaper around the waist of the wearer. The adhesive area may optionally be protected by a release tab which is peeled away when the ear is to be applied to the landing zone. In use, the child-carer positions the diaper around the body of the wearer, pulls the ear into position, releasing the release layer if present, and applies the ear to the landing zone to fit the diaper to the child or baby. In some cases the diaper, in particular the ear, may be damaged or torn during this process. Such a failure would be considered as a quality incident.

According to the present invention, consumer feedback would be solicited from the consumer, in this case the child-carer, by means of a report of the failure, i.e. ear torn or damaged, submitted back to the manufacturer in combination with the unique identifier associated with the diaper in question.

In order to maximize the data obtained, the child-carer may be asked to report the nature and extent of the failure, for example by comparing the diaper against a series of images which depict failure of the ear in different positions, and to different extent, and whereby the child-carer identifies the image which most closely depicts the failure experienced. Such a series of images could be presented in a questionnaire, but preferably could be presented by means of a web-based interface on a computer, tablet or other mobile device.

According to the present invention, the manufacturer correlates process data which has been captured and recorded during manufacture and consumer feedback. This can be achieved by using the unique identifier associated with the specific, individual diaper. By repeating this process across multiple (e.g. dozens, hundreds, etc.) of reports of consumer feedback, the manufacturer can identify the most critical product and process quality attributes which contribute to quality incidents experienced by the end user.

## Claims

1. A method for redesigning one or more process parameters of a first manufactured absorbent article, in order to provide different process parameters of a second manufactured absorbent article, and manufacturing the second manufactured absorbent article, wherein the method comprises the steps of:
i) associating a unique identifier with individual first manufactured absorbent articles, or with groups of first manufactured absorbent articles;
ii) capturing and recording process data relating to the first manufactured absorbent articles;
iii) soliciting and recording consumer feedback relating to in use performance of the first manufactured absorbent articles, wherein the consumer feedback includes data relating to the separation of back ears from diapers while under tension, leakage, or absorbent article fit;
iv) correlating, by a controller, the consumer feedback with process data of a specific, individual first manufactured absorbent article by means of the unique identifier;
v) determining different process parameters for a second manufactured absorbent article based on the correlated consumer feedback and process data for the first manufactured absorbent articles;
vi) applying, by the controller, the one or more different process parameters to a converting apparatus configured to manufacture absorbent articles; and
vii) manufacturing, by the converting apparatus, the second manufactured absorbent articles, the second manufactured absorbent articles being better adapted to meet consumer needs than the first manufactured absorbent article,
wherein each manufactured absorbent article is a diaper comprising: a topsheet; a backsheet; and an absorbent core placed between the topsheet and the backsheet to form a body of the diaper, and at least back ears which are attached to the body of the diaper in a waist region.

2. The method of claim 1 wherein the unique identifier is selected from the group consisting of serial numbers, bar codes, QR codes or combinations thereof.

3. The method of either of claims 1 or 2 wherein the process data is process performance data, preferably measurements, images, or combinations thereof.

4. The method of any of claims 1 to 3 wherein the in use performance data is recorded by means selected from the group consisting of questionnaire, sensor, image capture, audio recording, video recording or combinations thereof.

5. The method of claim 4 wherein the in use performance data is transmitted by means of written communication, by electronic communication, by verbal communication or by internet interface, or combinations thereof, preferably by means of telephone; or mobile devices, such as mobile telephones, smartphones, tablets.

6. The method of any of claims 1 to 5 wherein quality information generated by correlating the process data to the consumer feedback is selected from the group consisting of quality control data, quality data standards, process control strategies, process causal relationships, prognostics, process design improvements, process models, or combinations thereof.

## Patentansprüche

1. Verfahren zum Umgestalten eines oder mehrerer Verfahrensparameter eines ersten hergestellten Absorptionsartikels, um unterschiedliche Verfahrensparameter eines zweiten hergestellten Absorptionsartikels bereitzustellen, und Herstellen des zweiten hergestellten Absorptionsartikels, wobei das Verfahren die folgenden Schritte umfasst:
i) Zuordnen eines eindeutigen Bezeichners zu individuellen ersten hergestellten Absorptionsartikeln oder zu Gruppen von ersten hergestellten Absorptionsartikeln;
ii) Erfassen und Aufzeichnen von Verfahrensdaten, die sich auf die ersten hergestellten Absorptionsartikel beziehen;
iii) Abfragen und Aufzeichnen von Verbraucherrückkopplung, die sich auf die gebräuchliche Leistung der ersten hergestellten Absorptionsartikel bezieht, wobei die Verbraucherrückkopplung Daten enthält, die sich auf die Trennung von rückseitigen Seitenlappen von Windeln unter Zugspannung, Leckage oder die Absorptionsartikelpassform beziehen;
iv) Korrelieren, durch einen Regler, der Verbraucherrückkopplung mit Verfahrensdaten eines spezifischen, individuellen ersten hergestellten Absorptionsartikels mittels des eindeutigen Bezeichners;
v) Bestimmen unterschiedlicher Verfahrensparameter für einen zweiten hergestellten Absorptionsartikel basierend auf den korrelierten Verbraucherrückkopplungs- und Verfahrensdaten für die ersten hergestellten Absorptionsartikel;
vi) Anwenden, durch den Regler, des einen oder der mehreren unterschiedlichen Verfahrensparameter auf einen Umwandlungsapparat, der konfiguriert ist, um Absorptionsartikel herzustellen; und
vii) Herstellen, durch den Umwandlungsapparat, der zweiten hergestellten Absorptionsartikel, wobei die zweiten hergestellten Absorptionsartikel besser konzipiert sind, um den Verbraucherbedarf zu erfüllen, als der erste hergestellte Absorptionsartikel,
wobei jeder hergestellte Absorptionsartikel eine Windel ist, umfassend: eine Oberschicht; eine Unterschicht; und einen Absorptionskern, der zwischen der Oberschicht und der Unterschicht platziert ist, um einen Körper der Windel zu bilden, und wenigstens rückseitige Seitenlappen, die an dem Körper der Windel in einem Taillenbereich angebracht sind.

2. Verfahren nach Anspruch 1, wobei der eindeutige Bezeichner aus der Gruppe ausgewählt ist, bestehend aus Eingangsnummern, Barcodes, QR-Codes oder Kombinationen davon.

3. Verfahren nach einem der Ansprüche 1 oder 2, wobei die Verfahrensdaten Verfahrensleistungsdaten, vorzugsweise Messungen, Bilder oder Kombinationen davon, sind.

4. Verfahren nach einem der Ansprüche 1 bis 3, wobei die gebräuchlichen Leistungsdaten durch Mittel aufgezeichnet werden, die aus der Gruppe ausgewählt sind, bestehend aus Fragebogen, Sensor, Bilderfassung, Audioaufzeichnung, Videoaufzeichnung oder Kombinationen davon.

5. Verfahren nach Anspruch 4, wobei die gebräuchlichen Leistungsdaten mittels einer geschriebenen Kommunikation durch elektronische Kommunikation, durch verbale Kommunikation oder durch eine Internetschnittstelle oder Kombinationen davon, vorzugsweise mittels Telefon; oder mobilen Geräten, wie Mobiltelefone, Smartphones, Tablets übermittelt werden.

6. Verfahren nach einem der Ansprüche 1 bis 5, wobei Qualitätsinformationen, die durch Korrelieren der Verfahrensdaten mit der Verbraucherrückkopplung erzeugt werden, aus der Gruppe ausgewählt sind, bestehend aus Qualitätskontrolldaten, Qualitätsdatenstandards, Verfahrenssteuerungsstrategien, Verfahrenskausalbeziehungen, Prognosen, Verfahrensausführungsverbesserungen, Verfahrensmodellen oder Kombinationen davon.

## Revendications

1. Procédé de reconception d'un ou plusieurs paramètres de processus d'un premier article absorbant fabriqué, afin de fournir différents paramètres de processus d'un second article absorbant fabriqué, et de fabrication du second article absorbant fabriqué, dans lequel le procédé comprend les étapes de :
i) association d'un identifiant unique à des premiers articles absorbants fabriqués individuels, ou à des groupes de premiers articles absorbants fabriqués ;
ii) capture et enregistrement de données de processus relatives aux premiers articles absorbants fabriqués ;
iii) sollicitation et enregistrement d'une rétroaction de consommateur relative à une performance lors de l'utilisation des premiers articles absorbants fabriqués, dans lequel la rétroaction de consommateur comporte des données relatives à la séparation de pattes arrière de couches pendant une tension, une fuite ou un ajustement d'article absorbant ;
iv) corrélation, par un dispositif de commande, de la rétroaction de consommateur avec des données de processus d'un premier article absorbant fabriqué spécifique individuel au moyen de l'identifiant unique ;
v) détermination de différents paramètres de processus pour un second article absorbant fabriqué sur la base de la rétroaction de consommateur et des données de processus corrélées pour les premiers articles absorbants fabriqués ;
vi) application, par le dispositif de commande, des un ou plusieurs paramètres de processus différents à un appareil de conversion configuré pour fabriquer des articles absorbants ; et
vii) fabrication, par l'appareil de conversion, des seconds articles absorbants fabriqués, les seconds articles absorbants fabriqués étant mieux adaptés à la satisfaction des besoins du consommateur que le premier article absorbant fabriqué,
dans lequel chaque article absorbant fabriqué est une couche comprenant : une feuille de dessus ; une feuille de fond ; et une âme absorbante placée entre la feuille de dessus et la feuille de fond pour former un corps de la couche, et au moins des pattes arrière qui sont fixées au corps de la couche au niveau de la taille.

2. Procédé selon la revendication 1, dans lequel l'identifiant unique est sélectionné dans le groupe constitué par des numéros de série, des codes à barres, des codes QR ou des combinaisons de ceux-ci.

3. Procédé selon la revendication 1 ou 2, dans lequel les données de processus sont des données de performance de processus, de préférence des mesures, des images, ou des combinaisons de celles-ci.

4. Procédé selon l'une quelconque des revendications 1 à 3, dans lequel les données de performance lors de l'utilisation sont enregistrées par des moyens sélectionnés dans le groupe constitué par un questionnaire, un capteur, une capture d'image, un enregistrement audio, un enregistrement vidéo ou des combinaisons de ceux-ci.

5. Procédé selon la revendication 4 dans lequel les données de performance lors de l'utilisation sont transmises au moyen d'une communication écrite, d'une communication électronique, d'une communication verbale ou d'une interface Internet, ou des combinaisons de celles-ci, de préférence au moyen d'un téléphone ; ou des dispositifs mobiles, tels que des téléphones mobiles, des téléphones intelligents, des tablettes.

6. Procédé selon l'une quelconque des revendications 1 à 5 dans lequel des informations de qualité générées en corrélant les données de processus à la rétroaction de consommateur sont sélectionnées dans le groupe constitué par des données de contrôle de qualité, des normes de données de qualité, des stratégies de contrôle de processus, des relations causales de processus, des pronostics, des améliorations de conception de processus, des modèles de processus, ou des combinaisons de ceux-ci.
